# EUROPEAN PATENT APPLICATION

(11) **EP 4 082 589 A1**
(43) Date of publication of application: **02.11.2022**
(21) Application number: 20905023.6
(22) Date of filing: 11.12.2020
(51) Int. Cl.: A61M 5/14, A61M 25/09

(54) **WIRE DELIVERY DEVICE, AND MEDICINAL LIQUID INJECTING DEVICE**

(30) Priority: 25.12.2019 JP 2019234741
(71) Applicant: Asahi Intecc Co., Ltd., Seto-shi, Aichi 489-0071 (JP)
(72) Inventor: KUBO Yuta, Seto-shi, Aichi 489-0071 (JP)
(74) Representative: Winter, Brandl - Partnerschaft mbB
(86) International application number: PCT/JP2020/046254
(87) International publication number: WO 2021/131784

(57) **Abstract**

A wire delivery device for delivering a wire includes: a rod that has a long external shape and a side surface with concaves/convexes arranged along a longitudinal direction and is capable of moving in a distal end direction of the wire delivery device; a gear that engages with the concaves/convexes of the rod and rotates in response to the movement of the rod in the distal end direction; and a first roller and a second roller that sandwich the wire therebetween and individually rotate in response to the rotation of the gear to deliver the wire in the distal end direction of the wire delivery device.

## Description

### TECHNICAL FIELD

The present invention relates to a wire delivery device, and a drug solution injection device.

### BACKGROUND ART

There are known devices for injecting a drug solution into a patient's body. For example, Patent Literature 1 discloses an automatic injection device including a needle and first and second main body members, in which, when the first and second main body members reciprocate, the needle is exposed to inject a drug solution from a patient's body surface. For example, Patent Literature 2 discloses an injection device including a syringe cylinder, a plunger, an injection needle, and an elongate sheath, in which the injection needle is inserted into a living body lumen to inject a drug solution into a living tissue.

### CITATION LIST

### Patent Literature

Patent Literature 1: JP 2012-525869 W
Patent Literature 2: JP 2016-532496 W

### SUMMARY OF INVENTION

### TECHNICAL PROBLEM

There has been a desire to enable precise control of a drug solution injection dose because there are various doses for a drug solution to be injected into a patient's body. In this regard, for example, combination of a needle catheter having a puncture needle with a wire allows precise control of the drug solution injection dose. Specifically, in a state that the wire is inserted into the needle catheter, the wire is moved in a distal end direction to extrude the drug solution in the needle catheter and discharge the drug solution from the puncture needle. In this case, there has been a desire to control a movement amount of the wire in the needle catheter because the wire movement amount is proportional to a discharge volume of the drug solution from the puncture needle. In addition, there has been a desire to improve an efficiency of the wire movement amount relative to a manipulation quantity at hand in order to efficiently discharge the drug solution from the puncture needle. However, for the devices described in Patent Literatures 1 and 2, movement of the wire in the distal end direction in the needle catheter is not taken into consideration at all.

Such a problem is not limited to the wire delivery device for delivering the wire to the needle catheter configured to inject the drug solution, and is common to all wire delivery devices such as a wire delivery device for delivering the wire directly to a living body lumen, and a wire delivery device for delivering the wire to any medical device such as a catheter having no puncture needle.

The present invention has been made to solve at least some of the aforementioned problems, and an object of the present invention is to improve efficiency of a wire movement amount relative to a manipulation quantity at hand while controlling the wire movement amount in a wire delivery device for delivering the wire.

### SOLUTION TO PROBLEM

The present invention has been made to solve at least some of the aforementioned problems, and can be implemented as the following aspects.
(1) According to one aspect of the present invention present invention, a wire delivery device for delivering a wire. This wire delivery device includes: a rod that has a long external shape and a side surface with concaves/convexes arranged along a longitudinal direction and is capable of moving in a distal end direction of the wire delivery device; a gear that engages with the concaves/convexes of the rod and rotates in response to the movement of the rod in the distal end direction; and a first roller and a second roller that sandwich the wire therebetween and individually rotate in response to the rotation of the gear to deliver the wire in the distal end direction of the wire delivery device.
   According to this configuration, the wire delivery device includes the rod that is capable of moving in the distal end direction of the wire delivery device and has concaves/convexes arranged along the longitudinal direction; the gear that engages with the concaves/convexes of the rod and rotates; and the first roller and the second roller that individually rotate in response to the rotation of the gear to deliver the wire in the distal end direction. This makes it possible to provide a wire delivery device that can move the rod in the distal end direction of the wire delivery device and accordingly deliver the wire in the same distal end direction. As described above, when the rod movement direction and the wire delivery direction are the same (both are in the distal end direction of the wire delivery device), it is easy for an operator to intuitively grasp the wire delivery direction. According to this configuration, the wire movement amount (amount by which the wire is delivered) can be increased compared to the rod movement amount (amount by which the rod is pushed, in other words, manipulation quantity at hand) by changing the relationship between the concaves/convexes of the rod and the gear, so that the efficiency of the wire movement amount relative to the manipulation quantity at hand can be improved. Furthermore, according to this configuration, the wire movement amount can be proportional to a length of the concave/convex portion of the rod that has passed through the engagement portion with the gear, and therefore the wire movement amount can be controlled by controlling the rod movement amount (amount by which the rod is pushed, i.e. manipulation quantity at hand). As a result, it is possible to improve the efficiency of the wire movement amount relative to the manipulation quantity at hand while controlling the wire movement amount in the wire delivery device for delivering the wire.
(2) The wire delivery device according to the aforementioned aspect may be configured such that the rod can move not only in the distal end direction of the wire delivery device but also in a proximal end direction of the wire delivery device, and, in a first path in the distal end direction, the rod moves with the concaves/convexes engaging with the gear, and in a second path in the proximal end direction, the rod moves with the concaves/convexes not engaging with the gear.
   According to this configuration, the rod can move not only in the distal end direction of the wire delivery device but also in the proximal end direction of the wire delivery device. Thereby, even a long wire can be delivered by reciprocating the rod in the distal end direction and the proximal end direction of the wire delivery device. In the second path in the proximal end direction, the rod moves with the concaves/convexes not engaging with the gear, and therefore the wire that has been delivered from the wire delivery device can be prevented from turning back.
(3) The wire delivery device according to the aforementioned aspects may be configured such that the wire delivery device further includes a main body that accommodates a part on a distal end side of the rod, the gear, and the first roller and the second roller, in which the main body has an inner surface having a first groove portion for the first path, a second groove portion for the second path, a connection portion for connecting the first groove portion with the second groove portion, and the rod further has a protruding portion protruding on the side surface, and moves through the first path while the protruding portion engages with the first groove portion and moves through the second path while the protruding portion engages with the second groove portion.
   According to this configuration, the wire delivery device includes the main body, in which the first groove portion and second groove portion are formed on the inner surface of the main body, and the protruding portion is formed on the rod, to construct the first path through which the rod moves with the concaves/convexes engaging with the gear, and the second path through which the rod moves with the concaves/convexes not engaging with the gear.
(4) The wire delivery device according to the aforementioned aspects may be configured such that the main body accommodating the part on the distal end side of the rod, the gear, and the first roller and the second roller further has a connector that is attachable with a distal end portion of an accommodation member for the wire.
   According to this configuration, since the wire delivery device includes the main body having the connector attachable with the distal end portion of the wire accommodation member, a wire product can be directly attached to the connector of the main body and then used. In the wire product, the wire in a sterile condition is accommodated in the accommodation member. Thus, compared to a case where a wire is taken out to the outside from an accommodation member, this configuration makes it possible to eliminate a labor required for taking out the wire to improve the convenience, and reduce the risk of wire contamination to improve safety of the procedure.
(5) The wire delivery device according to the aforementioned aspects may further include a wire winding portion capable of winding up the wire while fixing a part on the proximal end side of the wire.
   According to this configuration, the wire delivery device includes the wire winding portion capable of winding up the wire, allowing the wire to be wound around and accommodated in the wire winding portion to improve convenience.
(6) One aspect of the present invention provides a drug solution injection device. This drug solution injection device includes: a catheter having a hollow first main body with a long external shape, a drug solution lumen that is formed inside the first main body to contain a drug solution; a wire that is inserted into the drug solution lumen and advanced from a proximal end toward a distal end of the drug solution lumen to discharge the drug solution in the drug solution lumen from a distal end of the catheter; and the wire delivery device that is configured as described above to deliver the wire.
   According to this configuration, the drug solution injection device includes the catheter having the drug solution lumen, the wire for discharging the drug solution in the drug solution lumen from the distal end of the catheter, and the wire delivery device. Thus, the volume by which the drug solution is discharged from the distal end of the catheter can be the same as the volume by which the wire is delivered into the drug solution lumen from the wire delivery device, so that the drug solution injection dose can be precisely controlled. In this configuration, the drug solution can be extruded by the wire to thoroughly use the drug solution in the drug solution lumen, reducing waste of the drug solution.
(7) The drug solution injection device according to the aforementioned aspect may be configured such that the wire has a first engagement portion on the proximal end portion and is accommodated in an accommodation member, and the accommodation member has a second engagement portion that engages with the first engagement portion on the distal end portion to restrict the movement of the wire in the distal end direction.
   According to this configuration, the wire has the first engagement portion on the proximal end portion. The accommodation member for accommodating the wire has the second engagement portion that engages with the first engagement portion on the distal end portion to restrict the movement of the wire in the distal end direction. Thus, the wire is sequentially delivered by the wire delivery device, and once the proximal end portion (first engagement portion) of the wire reaches the distal end portion (second engagement portion) of the accommodation member, the first engagement portion can engage with the second engagement portion to restrict the movement of the wire in the distal end direction (i.e. stop the delivery of the wire). As a result, the wire can be prevented from falling out of the wire accommodation member, so that convenience and safety can be improved.
(8) The drug solution injection device according to the aforementioned aspects may be configured such that the wire has an outer diameter substantially constant from the distal end to the proximal end, and the wire delivery device advances the wire inside the drug solution lumen to discharge the drug solution in a volume corresponding to an advanced length of the wire, from the distal end of the catheter.
   According to this configuration, the wire delivery device discharges the drug solution in a volume corresponding to the advanced length of the wire, from the distal end of the catheter. Thereby, the drug solution injection dose can be precisely controlled and waste of the drug solution can be reduced.
(9) The drug solution injection device according to the aforementioned aspects may be configured such that the catheter is a needle catheter that further has a hollow puncture needle disposed on a distal end portion of the first main body, and the wire discharges the drug solution in the drug solution lumen from a distal end of the puncture needle.
   According to this configuration, the catheter can be configured as the needle catheter having the puncture needle, so that the puncture needle can be used to puncture a living tissue and inject the drug solution into the living tissue.

The present invention can be achieved in various forms, for example, in a form of a wire delivery device, a drug solution injection device including the wire delivery device, a handle member including the wire delivery device and attached to a catheter/needle catheter/catheter for delivering the needle catheter, or the like, a system including the wire delivery device, a method for manufacturing these devices and system, or the like.

### BRIEF DESCRIPTION OF DRAWINGS

Fig. 1 is an explanatory diagram illustrating a configuration of a drug solution injection device according to the first embodiment.
Fig. 2 is an explanatory diagram illustrating a sectional configuration of a needle catheter.
Fig. 3 is an explanatory diagram illustrating a configuration of a wire delivery device.
Fig. 4 is an explanatory diagram illustrating a configuration of a main body.
Fig. 5 is an explanatory diagram illustrating a configuration of a gear and a first roller viewed from a direction A1 in Fig. 3.
Fig. 6 is an explanatory diagram illustrating a configuration of a rod viewed from a direction A2 in Fig. 3.
Fig. 7 is an explanatory diagram illustrating a configuration of a wire and an accommodation member.
Fig. 8 is a diagram for explaining delivery of the wire by the wire delivery device and discharge of a drug solution.
Fig. 9 is a diagram for explaining movement of the rod.
Fig. 10 is a diagram for explaining repetition of the wire delivery.
Fig. 11 is an explanatory diagram illustrating a configuration of a wire delivery device according to the second embodiment.
Fig. 12 is an explanatory diagram illustrating a configuration of a wire delivery device according to the third embodiment.
Fig. 13 is an explanatory diagram illustrating a configuration of a main body according to the fourth embodiment.
Fig. 14 is an explanatory diagram illustrating a configuration of a rod according to the fourth embodiment.
Fig. 15 is an explanatory diagram illustrating a configuration of a main body according to the fifth embodiment.
Fig. 16 is an explanatory diagram illustrating a configuration of a rod according to the fifth embodiment.
Fig. 17 is an explanatory diagram illustrating a configuration of a wire delivery device according to the sixth embodiment.
Fig. 18 is an explanatory diagram illustrating a configuration of a drug solution injection device according to the seventh embodiment.
Fig. 19 is an explanatory diagram illustrating a configuration of a drug solution injection device according to the eighth embodiment.

### EMBODIMENTS OF THE INVENTION

### <First Embodiment>

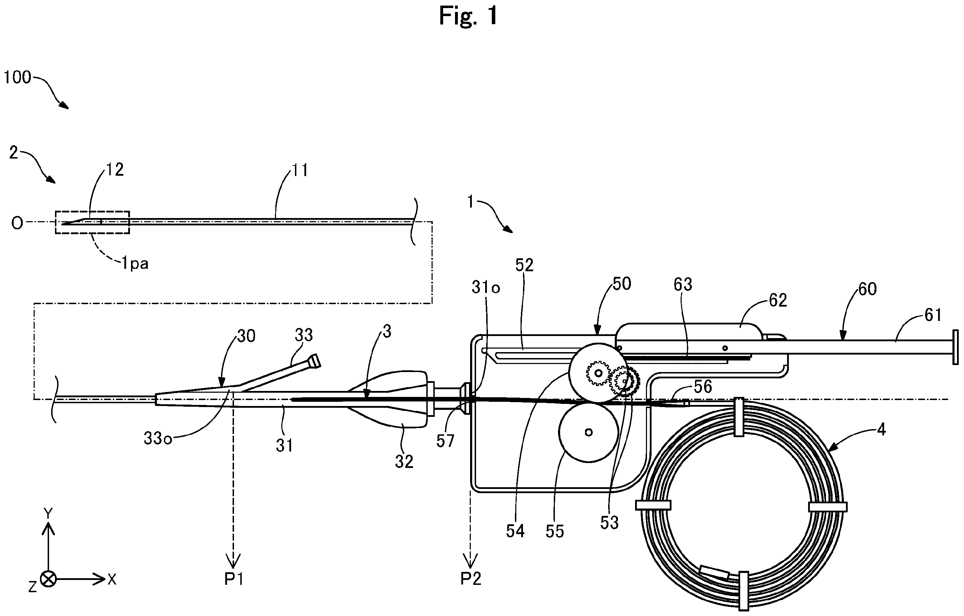

Fig. 1 is an explanatory diagram illustrating a configuration of a drug solution injection device 100 according to the first embodiment. The drug solution injection device 100 is used for injecting a drug solution into a patient's body using a needle catheter 2 that can be inserted into a living body lumen. Herein, the living body lumen includes various lumens such as a vascular system, a lymphatic gland system, a biliary system, a urinary tract system, a respiratory system, a digestive system, a secretory gland, and a reproductive organ. The drug solution injection device 100 includes a wire delivery device 1, a needle catheter 2, a wire 3, and an accommodation member 4.

In Fig. 1, an axis passing through the center of the needle catheter 2 is represented by an axis line O (dot and dash line). In the example in Fig. 1, the axis line O passes through the center of the needle catheter 2, but is different from an axis passing through each center of the wire delivery device 1, the wire 3, and the accommodation member 4. However, the axis line O may coincide with an axis passing through each center of the wire delivery device 1, the needle catheter 2, the wire 3, and the accommodation member 4. Fig. 1 illustrates axes X, Y, and Z orthogonal to each other. The axis X corresponds to a longitudinal direction (length direction) of the drug solution injection device 100 (wire delivery device 1, needle catheter 2, wire 3, and accommodation member 4), the axis Y corresponds to a height direction of the drug solution injection device 100, and the axis Z corresponds to a width direction of the drug solution injection device 100. The left side (-X axis direction) of Fig. 1 is referred to as a "distal end side" of the drug solution injection device 100 and each component, and the right side of Fig. 1 (+X axis direction) is referred to as a "proximal end side" of the drug solution injection device 100 and each component. As for the drug solution injection device 100 and each component, an end portion positioned on the distal end side is referred to as a "distal end", and the distal end and the vicinity thereof are referred to as a "distal end portion". In addition, an end portion located on the proximal end side is referred to as a "proximal end", and the proximal end and the vicinity thereof are referred to as a "proximal end portion". The distal end side is inserted into a living body, and the proximal end side is manipulated by an operator such as a surgeon. These definitions are common for the figures following Fig. 1.

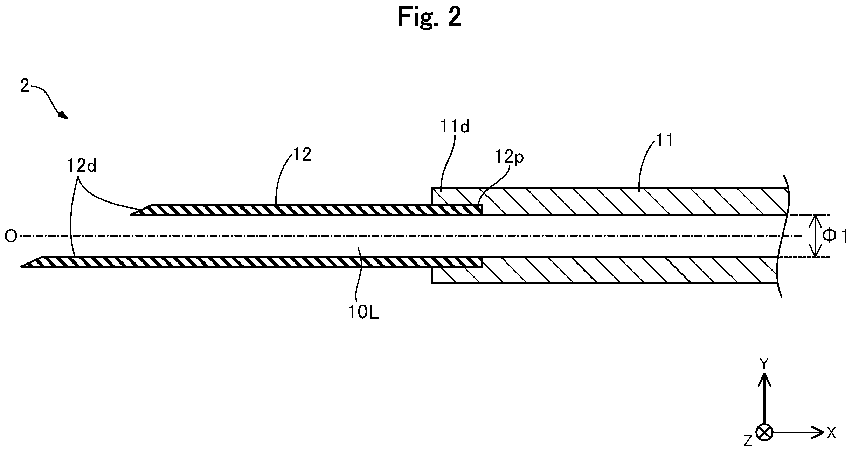

Fig. 2 is an explanatory diagram illustrating a sectional configuration of the needle catheter 2. Fig. 2 illustrates the sectional configuration of a part 1pa (Fig. 1: dashed frame) on the distal end side of the needle catheter 2. The needle catheter 2 is inserted into a living body lumen of a patient and used for transporting the drug solution to a desired position in the patient's body to inject the drug solution into the desired position in the patient's body. The needle catheter 2 includes a hollow shaft 11, a puncture needle 12, and a connector 30.

The hollow shaft 11 is a long member extending along the axis line O. The hollow shaft 11 has a hollow and almost cylindrical shape, in which openings are formed on each of a distal end portion 11d and a proximal end portion, and an inner cavity communicating between the both openings is formed thereinside. A coil body or a braided body may be embedded in a thick-walled portion of the hollow shaft 11 to improve at least some of flexibility, torquability, pushability, kink resistance, blood vessel followability, lesion passableness, and operability. The hollow shaft 11 may be composed of multiple layers made of same or different materials.

As illustrated in Fig. 2, the puncture needle 12 is a hollow needle in which openings are formed on each of a distal end portion 12d and a proximal end portion 12p, and an inner cavity communicating between the both openings is formed thereinside. In the puncture needle 12, a sharp needle tip is formed on the distal end portion 12d, and the proximal end portion 12p is joined to the distal end portion 11d of the hollow shaft 11. The joining of the puncture needle 12 can be carried out using any joining agent such as an epoxy adhesive. Herein, the puncture needle 12 is joined to the hollow shaft 11 while the inner cavity of the puncture needle 12 and the inner cavity of the hollow shaft 11 are in communication with each other. Both the inner cavity of the puncture needle 12 and the inner cavity of the hollow shaft 11 have a substantially constant inner diameter Φ1. The inner cavity of the puncture needle 12 and the inner cavity of the hollow shaft 11 constitute a drug solution lumen 10L that contains the drug solution. The drug solution lumen 10L has an almost circular transverse sectional shape.

As illustrated in Fig. 1, the connector 30 is connected to the proximal end portion of the hollow shaft 11 and used for feeding the drug solution to the needle catheter 2 and inserting the wire 3 into the needle catheter 2. The connector 30 includes a first extension portion 31, a blade portion 32, and a second extension portion 33.

The first extension portion 31 is an almost hollow cylindrical member connected to the proximal end portion of the hollow shaft 11 and extending along the axis line O. An inner cavity extending along the axis line O is formed inside the first extension portion 31. In the example of Fig. 1, in addition to the inner cavity of the puncture needle 12 and the inner cavity of the hollow shaft 11 described above, the inner cavity of the first extension portion 31 also constitutes the drug solution lumen 10L. A wire insertion port 31o communicating between the drug solution lumen 10L and the outside is formed on the proximal end portion of the first extension portion 31. The second extension portion 33 is an almost hollow cylindrical member extending from a side surface of the first extension portion 31 in a direction different from the first extension portion 34 on the distal end side of the first extension portion 31. An inner cavity in communication with the drug solution lumen 10L is formed inside the second extension portion 33. A drug solution feed port 33o communicating between the drug solution lumen 10L and the inner cavity of the second extension portion 33 is formed on the distal end portion of the second extension portion 33.

In the needle catheter 2 according to the first embodiment, a position P1 of the drug solution feed port 33o is positioned on the distal end side with respect to a position P2 of the wire insertion port 31o. The blade portion 32 is composed of two blade-shaped members extending from the side surface of the first extension portion 31 in the ±Y-axis direction on the proximal end side of the first extension portion 31. The blade portion 32 is used when the operator grips the connector 30. At least some members of the first extension portion 31, the blade portion 32, and the second extension portion 33, which constitute the connector 30 may be integrally configured.

Preferably, the hollow shaft 11 has antithrombogenicity, flexibility, and biocompatibility, and can be made of a resin material or a metal material. As the resin material, for example, a polyamide resin, a polyolefin resin, a polyester resin, a polyurethane resin, a silicon resin, a fluororesin, or the like can be adopted. As the metal material, for example, a stainless steel such as SUS304, an Ni-Ti alloy, a cobalt-chromium alloy, or the like can be used. Preferably, the puncture needle 12 has antithrombogenicity and biocompatibility, and can be made of e.g. a metal material of a stainless steel such as SUS304, an Ni-Ti alloy, a cobalt-chromium alloy, or the like. The connector 30 can be made of a resin material, e.g. a polyurethane, a polypropylene, a hard polyvinyl chloride, or the like.

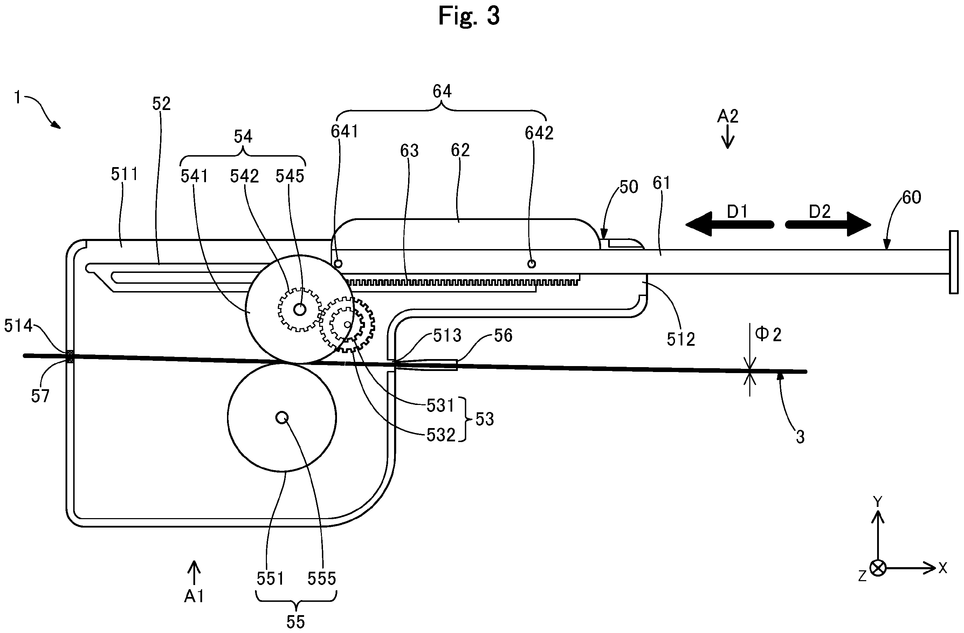

Fig. 3 is an explanatory diagram illustrating a configuration of the wire delivery device 1. The wire delivery device 1 is intended to deliver the wire 3 to the drug solution lumen 10L of the needle catheter 2. The wire delivery device 1 includes a main body 50, a gear 53, a first roller 54, a second roller 55, a connector 56, and a rod 60. In Fig. 3, for convenience of illustration, the main body 50 is represented only by its outline as a transparent member, and each member accommodated in the main body 50 is represented by solid lines. Also, in Fig. 3, a third gear 542, a part of a first gear 531, and a part of a second gear 532 which are originally invisible because they are hidden under a roller portion 541 are represented by thin solid lines.

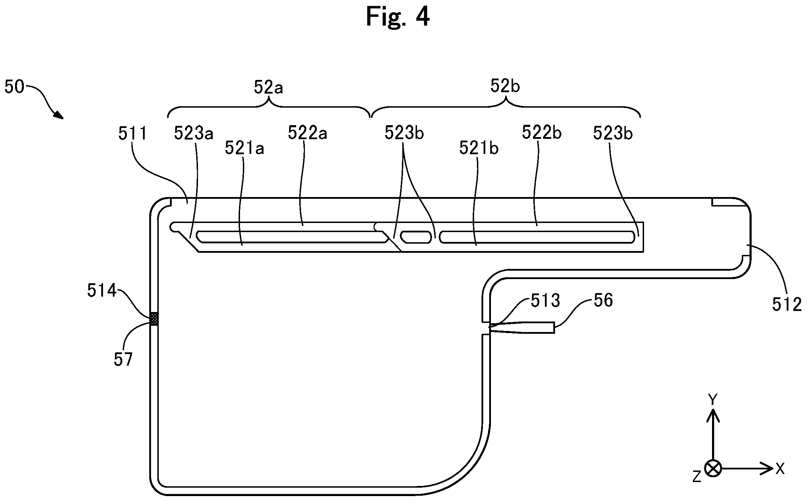

Fig. 4 is an explanatory diagram illustrating a configuration of the main body 50. The main body 50 is a chassis that accommodates the gear 53, the first roller 54, the second roller 55, and a part on the distal end side of the rod 60 thereinside (in the inside of the main body 50). The main body 50 viewed from the top has an almost rectangular shape with an extension portion partially extending in the +X-axis direction, as illustrated in Fig. 4. The main body 50 has four openings 511 to 514 and a groove portion 52.

The opening 511 is a long rectangular through hole formed on the +Y-axis direction side wall of the main body 50. An after-mentioned projection portion 62 of the rod 60 protrudes from the opening 511. The opening 512 is an almost rectangular through hole formed on the +X-axis direction side wall of the extension portion of the main body 50. An after-mentioned rod main body 61 of the rod 60 protrudes from the opening 512. The opening 513 is an almost circular through hole formed on the +X-axis direction side wall of the main body 50. The hollow connector 56 is disposed on the opening 513. The connector 56 is joined to the main body 50 so as to communicate between the opening 513 of the main body 50 and an inner cavity of the connector 56. For the joining, any joining agent such as an epoxy adhesive can be used. The connector 56 has a reduced-diameter shape with an outer diameter and an inner diameter reduced from the proximal end side to the distal end side, to which an after-mentioned distal end member 43 of the accommodation member 4 can be attached (inserted). The opening 514 is an almost circular through hole formed on the -X-axis direction side wall of the main body 50. A sealing member 57 is disposed on the opening 514. The sealing member 57 is made of an elastic body, to seal the circumference of the wire 3.

The groove portion 52 is formed on the inner surface of the main body 50. The groove portion 52 has first groove portions 521a and 521b, second groove portions 522a and 522b, and connection portions 523a and 523b. The first groove portions 521a and 521b are located on a side closer to the gear 53 (in the example in Fig. 4, in the -Y-axis direction) with respect to the second groove portions 522a and 522b, and extend in the longitudinal direction (X-axis direction) of the wire delivery device 1. The second groove portions 522a and 522b are located on a side farther from the gear 53 (in the example in Fig. 4, in the +Y-axis direction) with respect to the first groove portions 521a and 521b, and extend parallel to the first groove portions 521a and 521b in the longitudinal direction (X-axis direction) of the wire delivery device 1. The connection portions 523a and 523b connect the first groove portions 521a and 521b with the second groove portions 522a and 522b on a total of four positions, the distal end position, the proximal end position, and two middle positions. Herein, in the groove portion 52, the first groove portion 521a, the second groove portion 522a, and the connection portion 523a with the subscript "a" are deeper than the first groove portion 521b, the second groove portion 522b, and the connection portion 523b with the subscript "b". In other words, the groove portion 52a on the distal end side is shallower than the groove portion 52b on the proximal end side.

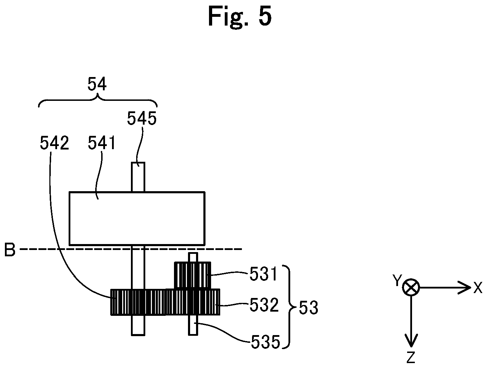

Fig. 5 is an explanatory diagram illustrating a configuration of the gear 53 and the first roller 54 viewed from the direction A1 in Fig. 3. The gear 53 has the first gear 531 and the second gear 532 that are fixed to a shaft 535. The first gear 531 is located above the second gear 532 (in other words, on the side farther from the inner surface of the main body 50) and has a smaller diameter than of the second gear 532. The first gear 531 engages with an after-mentioned concave/convex portion 63 of the rod 60. The second gear 532 is located under the first gear 531 (in other words, on the inner surface side of the main body 50) and has a larger diameter than of the first gear 531. The second gear 532 engages with a third gear 542 of the first roller 54. The first gear 531 and the second gear 532 rotate about the shaft 535.

The first roller 54 has the roller portion 541 and the third gear 542 gear that are fixed to a shaft 545. The roller portion 541 is almost hollow cylindrical or almost columnar. The roller portion 541 is located above the third gear 542 (in other words, on the side farther from the inner surface of the main body 50). The roller portion 541 and the third gear 542 rotate about the shaft 545. At the position indicated by a dashed line B in Fig. 5, a compartment portion for preventing the wire 3 from dropping out in the Z-axis direction may be disposed. As illustrated in Fig. 3, the second roller 55 has a roller portion 551 fixed to a shaft 555. The roller portion 551 is arranged parallel to the roller portion 541 such that an outer peripheral surface of the roller portion 551 is in contact with the roller portion 541 or leaves a gap allowing the wire 3 to be sandwiched therebetween. The roller portion 551 rotates about the shaft 555.

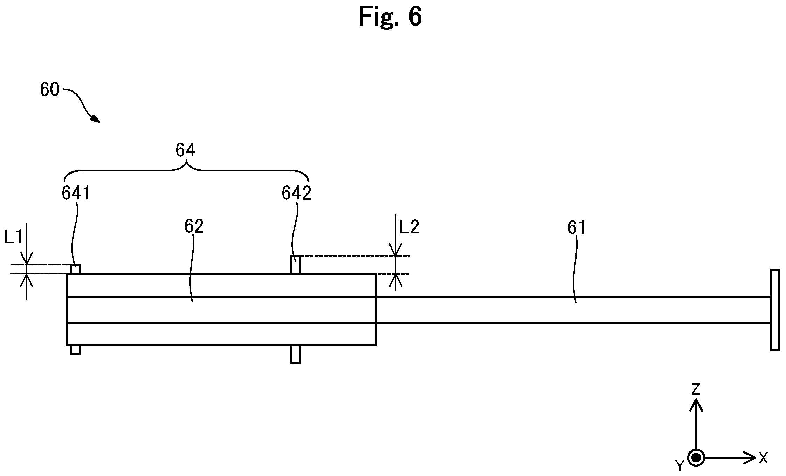

Fig. 6 is an explanatory diagram illustrating a configuration of the rod 60 viewed from the direction A2 in Fig. 3. The rod 60 has a long external shape that extends in the longitudinal direction (X-axis direction) of the wire delivery device 1. As illustrated in Fig. 3, the rod 60 has the rod main body 61, the projection portion 62, the concave/convex portion 63, and a protruding portion 64. The rod main body 61 has a long and almost quadrangular prismatic shape that extends in the longitudinal direction (X-axis direction) of the wire delivery device 1. The projection portion 62 is a portion where the +Y-axis direction side wall of the rod main body 61 partially protrudes, and is disposed on the distal end side of the rod main body 61. The concave/convex portion 63 is a long and teeth-shaped member that extends in the longitudinal direction (X-axis direction) of the wire delivery device 1. The concave/convex portion 63 is fixed to a -Y-axis direction side wall (side surface) of the rod main body 61 (in other words, opposite side to the projection portion 62 side). Similarly to the projection portion 62, the concave/convex portion 63 is disposed on the distal end side of the rod main body 61.

As illustrated in Fig. 6, a protruding portion 64 is provided so as to protrude in both directions of the ±Z-axis on the side wall (side surface) of the rod main body 61. The protruding portion 64 has a distal end-side protruding portion 641 disposed on the distal end portion of the rod main body 61, and a proximal end-side protruding portion 642 disposed on the proximal end side with respect to the distal end-side protruding portion 641. Herein, a protruding length L1 of the distal end-side protruding portion 641 (in other words, length from the side surface of the rod main body 61) is shorter than a protruding length L2 of the proximal end-side protruding portion 642. The protruding length L1 is substantially equal to the depth of the groove portion 52a on the distal end side, and the protruding length L2 is substantially equal to the depth of the groove portion 52b on the proximal end side.

As illustrated in Fig. 3, a part on the distal end side of the rod 60 is accommodated in the main body 50, and the remaining part on the proximal end side protrudes outward from the opening 512 of the main body 50. At this time, the projection portion 62 of the rod 60 protrudes outward from the opening 511 of the main body 50. In addition, the protruding portion 64 of the rod 60 (distal end-side protruding portion 641, proximal end-side protruding portion 642) engages with the groove portion 52. The rod 60 is moved while engaging the protruding portion 64 with the groove portion 52, so that the rod 60 can moves both in a distal end direction D1 and a proximal end direction D2 of the wire delivery device 1.

The main body 50, the gear 53, main body parts of the first roller 54 and the second roller 55 excluding the outer peripheral surfaces, the connector 56, and the rod 60 can be made of a resin material, e.g. polyurethane, polypropylene, rigid polyvinyl chloride, or the like. The main body 50, the gear 53, the first roller 54 and the second roller 55, the connector 56, and the rod 60 may be made of the same material, or at least partially made of different materials. The sealing member 57, and the outer peripheral surfaces of the first roller 54 and the second roller 55 can be made of e.g. an elastic body such as a silicon rubber and a urethane rubber.

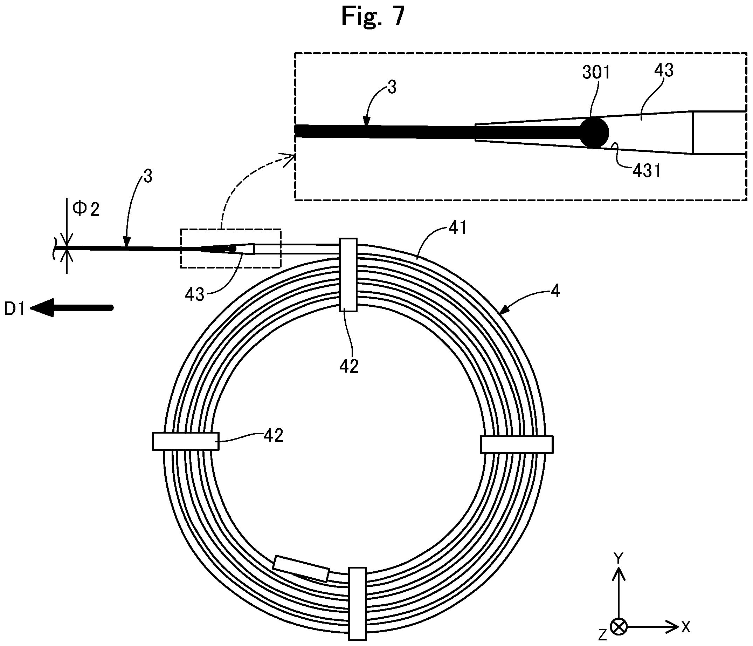

Fig. 7 is an explanatory diagram illustrating a configuration of the wire 3 and the accommodation member 4. Fig. 7 illustrates the wire 3 and the accommodation member 4 in a state that the wire 3 is sequentially delivered by the wire delivery device 1, and the proximal end portion of the wire 3 reaches the distal end portion of the accommodation member 4. In addition, an enlarged view illustrating the distal end portion of the accommodation member 4 is presented in the upper part of Fig. 7 (within the dashed frame).

The wire 3 is a long member having an almost circular transverse section. The wire 3 has a substantially constant outer diameter Φ2 from the distal end to the proximal end. The outer diameter Φ2 of the wire 3 is 80% or higher of the inner diameter Φ1 of the drug solution lumen 10L of the needle catheter 2 (Fig. 2). A first engagement portion 301 is formed on the proximal end portion of the wire 3. The first engagement portion 301 is a spherical body with a diameter thicker than the outer diameter Φ2 of the wire 3. For the wire 3, various forms can be adopted. For example, the wire 3 can be composed of a core shaft tapering from the proximal end side to the distal end side, and a coil body covering the distal end side of the core shaft. The wire 3 need not have the coil body, and the surface of the wire 3 may have a coating layer.

The accommodation member 4 has an accommodation tube 41, four fasteners 42, and the distal end member 43. The accommodation tube 41 is a tubular body longer than the wire 3 and accommodates the wire 3 thereinside (in the inside of the accommodation tube 41). The accommodation tube 41 is wound in a coil shape. Each of the four fasteners 42 engages with the outer peripheral surface of the accommodation tube 41 at four positions of the accommodation tube 41 to keep the coil shape of the accommodation tube 41. The distal end member 43 is a hollow member attached to the distal end of the accommodation tube 41. The distal end member 43 has a reduced-diameter shape with an outer diameter and an inner diameter reduced from the proximal end side to the distal end side. The wire 3 is sequentially delivered by the wire delivery device 1, and once the first engagement portion 301 of the wire 3 reaches the distal end member 43 of the accommodation member 4, the first engagement portion 301 of the wire 3 engages with an inner peripheral surface 431 of the accommodation member 4 (upper part of Fig. 7). Thereby, the accommodation member 4 can restrict the wire 3 from further moving in the distal end direction. As described above, the inner peripheral surface 431 of the accommodation member 4 functionally saves as a "second engagement portion".

Preferably, the core shaft of the wire 3 has antithrombogenicity, flexibility, and biocompatibility, and can be made of e.g. a metal material of a stainless steel such as SUS304, an Ni-Ti alloy, or the like. The coil body of the wire 3 can be made of e.g. a stainless steel alloy such as SUS304 and SUS316, a superelastic alloy such as Ni-Ti alloy, a piano wire, a radiolucent alloy such as nickel-chromium alloy and cobalt alloy, gold, platinum, tungsten, or a radiopaque alloy such as an alloy containing any of these elements (e.g. a platinum-nickel alloy). The accommodation tube 41 of the accommodation member 4 can be made of a flexible material such as polyethylene, polypropylene, polyolefin, polyvinyl chloride, polystyrene, polyamide, and polyimide. The fastener 42 and the distal end member 43 of the accommodation member 4 can be made of a flexible material such as polypropylene, polyethylene, and polyacetal.

A method of using the drug solution injection device 100 will be explained. First, the operator prepares the wire delivery device 1 by inserting the distal end side of the wire 3 into the wire delivery device 1, as illustrated in Fig. 1. Specifically, the operator draws out a part on the distal end side of the wire 3 from the accommodation member 4 to insert the distal end side of the wire 3 into the wire delivery device 1 from the connector 56, so that the wire 3 is sandwiched between the first roller 54 and the second roller 55 and then drawn outside through the opening 514. In this state, the accommodation member 4 is fixed to the connector 56.

When using the drug solution injection device 100 along with an endoscope, the operator inserts the needle catheter 2 into the endoscope to move the puncture needle 12 of the needle catheter 2 to a target position (position to which the drug solution is administered) in a patient's body. Subsequently, the operator attaches the wire delivery device 1 to the wire insertion port 31o of the needle catheter 2 (Fig. 1). While the operator attaches the wire delivery device 1, the distal end portion of the wire 3 is inserted into the drug solution lumen 10L of the needle catheter 2. Then, the operator feeds the drug solution from the drug solution feed port 33o of the needle catheter 2. For example, a syringe containing the drug solution is attached to the proximal end portion of the second extension portion 33 to feed the drug solution from the syringe. The drug solution fed from the drug solution feed port 33o through the second extension portion 33 is charged into the drug solution lumen 10L of the needle catheter 2. Subsequently, the operator punctures the target position in the patient's body with the puncture needle 12.

When using the drug solution injection device 100 using no endoscope, the operator attaches the wire delivery device 1 to the needle catheter 2 to insert the needle catheter 2 into a patient's living body lumen while the drug solution is previously charged into the drug solution lumen 10L of the needle catheter 2. Then, the operator moves the puncture needle 12 of the needle catheter 2 to the target position in the patient's body to puncture the target position with the puncture needle 12. A guiding catheter or the like may be used to move the needle catheter 2.

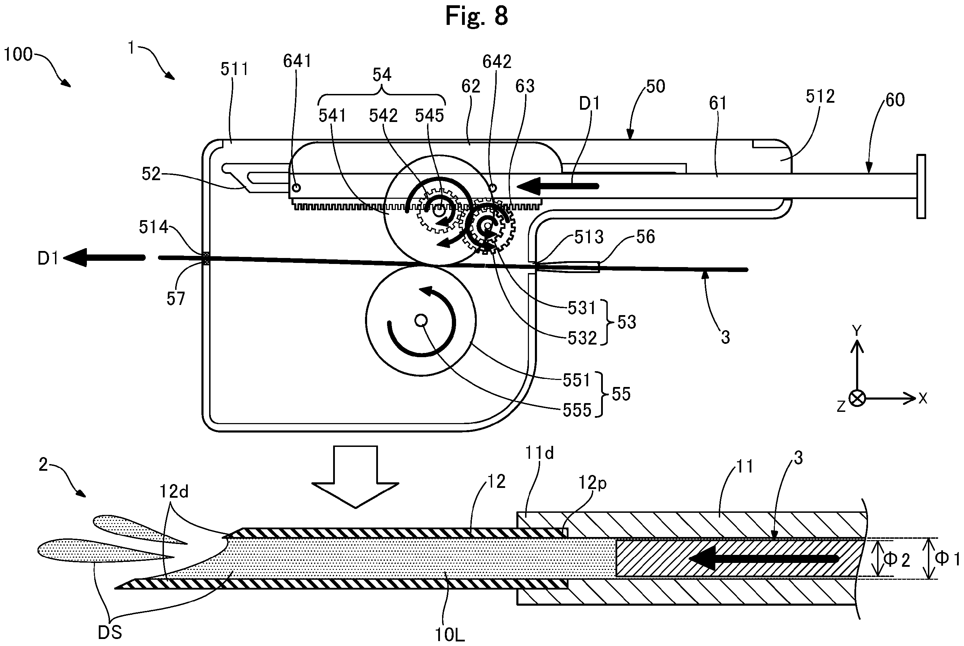

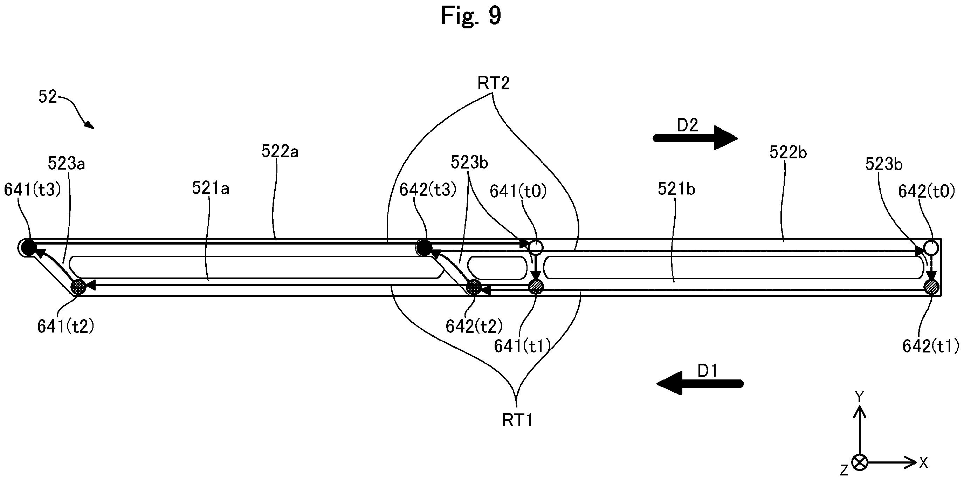

Fig. 8 is a diagram for explaining the delivery of the wire 3 by the wire delivery device 1 and the discharge of the drug solution. Fig. 9 is a diagram for explaining the movement of the rod 60. In Fig. 8, the wire delivery device 1 is illustrated on the upper part, and a part on the distal end side of the needle catheter 2 is illustrated on the lower part. In the upper part of Fig. 8, the rod 60, the third gear 542, a part of the first gear 531, and a part of the second gear 532 which are originally invisible because they are hidden under the roller portion 541 are represented by thin solid lines. Fig. 9 illustrates the groove portion 52 of the main body 50, and the protruding portion 64 of the rod 60 (distal end-side protruding portion 641, proximal end-side protruding portion 642). In Fig. 9, a moving path of the distal end-side protruding portion 641 is represented by a solid line, and a moving path of the proximal end-side protruding portion 642 is represented by a dashed line.

After puncturing the target position with the puncture needle 12, the operator moves the rod 60 in the distal end direction D1 of the wire delivery device 1, as illustrated in the upper part of Fig. 8. Herein, as illustrated in Fig. 9, in the groove portion 52 of the wire delivery device 1, an initial position of the protruding portion 64 (distal end-side protruding portion 641, proximal end-side protruding portion 642) of the rod 60 is on the proximal end portion of the second groove portion 522b (Fig. 9: t0). First, the operator pushes the rod 60 downward (in the -Y-axis direction) to move the protruding portion 64 of the rod 60 to the proximal end portion of the first groove portion 521b (Fig. 9: t1) while the protruding portion 64 engages with the connection portion 523b.

Then, the operator pushes the rod 60 in the distal end direction D 1 (in the -X-axis direction) to move the protruding portion 64 of the rod 60 to the distal end portions of the first groove portions 521a and 521b (Fig. 9: t2) while the protruding portion 64 engages with the first groove portions 521a and 521b. Thus, the path through which the protruding portion 64 of the rod 60 moves in the distal end direction D1 while engaging with the first groove portions 521a and 521b is also referred to as a "first path RT1". As illustrated in the upper part of Fig. 8, in the first path RT1, the concave/convex portion 63 of the rod 60 engages with the first gear 531 of the gear 53, so that the first gear 531 and the second gear 532 of the gear 53 individually rotates. In other words, the concave/convex portion 63 of the rod 60 functionally serves as a rack in a rack-and-pinion mechanism, and the first gear 531 of the gear 53 functionally serves as a pinion.

In response to the rotation of the second gear 532 of the gear 53, the third gear 542 engaging with the second gear 532 and the first roller 54 (roller portion 541) fixed coaxially with the third gear 542 individually rotate. When the second roller 55 (roller portion 551) rotates in association with the rotation of the first roller 54, the wire 3 sandwiched between the first roller 54 and the second roller 55 is delivered in the distal end direction D1. In this way, the operator moves the rod 60 in the distal end direction D1 to deliver (advance) the wire 3 in the distal end direction D1 from the wire delivery device 1. Then, as illustrated in the lower part of Fig. 8, a drug solution DS charged in the drug solution lumen 10L of the needle catheter 2 is extruded by the advanced wire 3, and the drug solution DS in an amount corresponding to an advanced volume of the wire 3 is discharged from the distal end of the puncture needle 12. That means, the drug solution DS in an amount corresponding to a movement amount of the rod 60 is discharged from the distal end of the puncture needle 12.

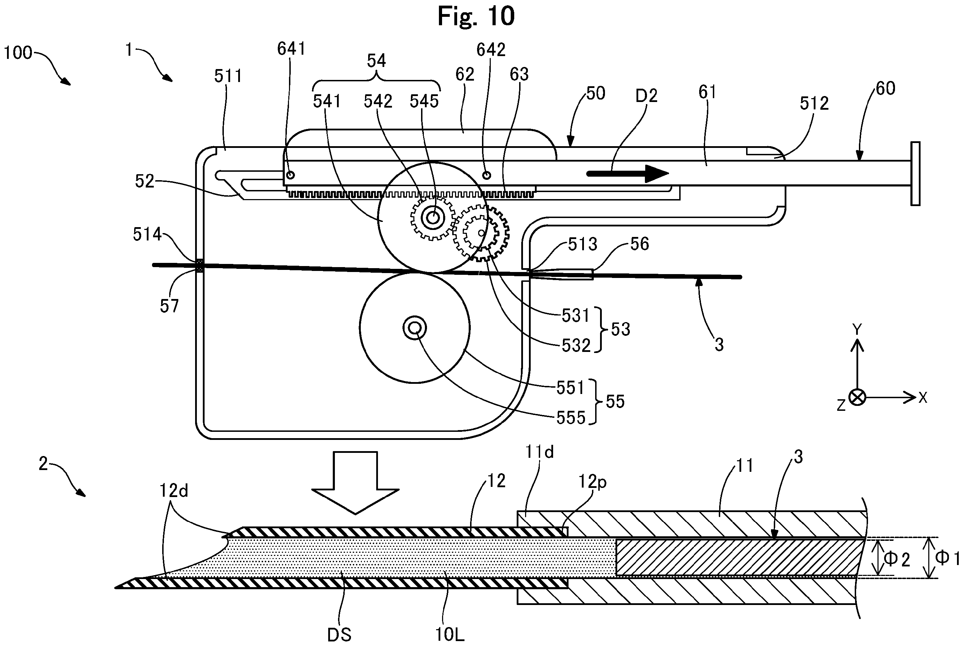

Fig. 10 is a diagram for explaining repetition of the delivery of the wire 3. Similarly to Fig. 8, the wire delivery device 1 is illustrated in the upper part of Fig. 10, and a part on the distal end side of the needle catheter 2 is illustrated in the lower part. In Fig. 10, the constituents which are originally invisible because they are hidden under the roller portion 541 are represented by thin solid lines. After advancing the rod 60 to the distal end portion, the operator pushes the rod 60 upward (in the +Y-axis direction) to move the protruding portion 64 (distal end-side protruding portion 641, proximal end-side protruding portion 642) of the rod 60 to the distal end portions of the second groove portions 522a and 522b (Fig. 9: t3) while the protruding portion 64 engages with the connection portions 523a and 523b.

Then, the operator draws back the rod 60 in the proximal end direction D2 (in the +X-axis direction) to move the protruding portion 64 of the rod 60 to the proximal end portions of the second groove portions 522a and 522b (Fig. 9: t0) while the protruding portion 64 engages with the second groove portions 522a and 522b. Thereby, the protruding portion 64 of the rod 60 returns to its initial position. Thus, the path through which the protruding portion 64 of the rod 60 moves in the proximal end direction D2 while engaging with the second groove portions 522a and 522b is also referred to as a "second path RT2". In the second path RT2, the concave/convex portion 63 of the rod 60 does not engage with the first gear 531 of the gear 53 as illustrated in the upper part of Fig. 10. For this reason, when the rod 60 moves through the second path RT2, the gear 53, the first roller 54, and the second roller 55 do not rotate, and the wire 3 does not move. Thus, when the rod 60 moves through the second path RT2, the drug solution DS is not discharged (lower part of Fig. 10).

As described above, the drug solution injection device 100 according to the first embodiment allows the operator to repeat a manipulation of moving the rod 60 described in Fig. 8 in the distal end direction D1 (in other words, pushing the rod 60 to the distal end portion) and a manipulation of moving the rod 60 described in Fig. 10 in the proximal end direction D2 (in other words, drawing back the rod 60 to the proximal end portion), to discharge the drug solution DS from the puncture needle 12.

As described above, the wire delivery device 1 according to the first embodiment includes: the rod 60 that is capable of moving in the distal end direction D1 of the wire delivery device 1 and has the concave/convex portion 63 arranged along the longitudinal direction (X-axis direction); the gear 53 (first gear 531) that engages with the concave/convex portion 63 of the rod 60 and rotates; and the first roller 54 and the second roller 55 that individually rotate in response to the rotation of the gear 53 (second gear 532) to deliver the wire 3 in the distal end direction D1 (Fig. 8). This makes it possible to provide the wire delivery device 1 that can move the rod 60 in the distal end direction of the wire delivery device 1 and accordingly deliver the wire 3 in the same distal end direction D1. As described above, when the movement direction of the rod 60 and the delivery direction of the wire 3 are the same (both are in the distal end direction D1 of the wire delivery device 1), it is easy for the operator to intuitively grasp the delivery direction of the wire 3. The wire delivery device 1 according to the first embodiment makes it possible to increase the movement amount of the wire 3 (amount by which the wire 3 is delivered) compared to the movement amount of the rod 60 (amount by which the rod 60 is pushed, in other words, manipulation quantity at hand) by changing the relationship between the concave/convex portion 63 of the rod 60 and the gear 53 (first gear 531, second gear 532), so that the efficiency of the wire movement amount relative to the manipulation quantity at hand can be improved. Furthermore, by the wire delivery device 1 according to the first embodiment, the movement amount of the wire 3 can be proportional to a length of the concave/convex portion 63 of the rod 60 that has passed through the engagement portion with the gear 53 (first gear 531), and therefore the movement amount of the wire 3 can be controlled by controlling the movement amount of the rod 60 (amount by which the rod 60 is pushed, i.e. manipulation quantity at hand). As a result, it is possible to improve the efficiency of the wire movement amount relative to the manipulation quantity at hand while controlling the movement amount of the wire 3 in the wire delivery device 1 for delivering the wire 3.

The wire delivery device 1 according to the first embodiment allows the rod 60 to move not only in the distal end direction D 1 of the wire delivery device 1 but also in the proximal end direction D2 of the wire delivery device 1 (Fig. 10). Thereby, even a long wire 3 can be delivered by reciprocating the rod 60 in the distal end direction D1 and the proximal end direction D2 of the wire delivery device 1. In the second path RT2 in the proximal end direction D2, since the rod 60 moves with the concave/convex portion 63 not engaging with the gear 53 (first gear 531), the wire 3 that has been delivered from the wire delivery device 1 can be prevented from turning back.

Furthermore, the wire delivery device 1 according to the first embodiment includes a main body 50. The first groove portions 521a and 521b and the second groove portions 522a and 522b are formed on the inner surface of the main body 50, and the protruding portion 64 (distal end-side protruding portion 641, proximal end-side protruding portion 642) are formed on the rod 60. This makes it easy to construct both the first path RT1 through which the rod 60 moves with the concave/convex portion 63 engaging with the gear 53 (first gear 531), and the second path RT2 through which the rod 60 moves with the concave/convex portion 63 not engaging with the gear 53 (first gear 531) (Fig. 8 to Fig. 10).

Furthermore, the wire delivery device 1 according to the first embodiment includes the main body 50 having the connector 56 attachable with the distal end portion (distal end member 43) of the accommodation member 4 of the wire 3. Thereby, a commercially available wire product can be attached directly to the connector 56 of the main body 50 and used. As described in Fig. 7, in the wire product, the wire 3 in a sterile condition is accommodated in the accommodation member 4. Thus, compared to the case where the wire 3 is thoroughly taken out to the outside from the accommodation member 4, the wire delivery device 1 according to the first embodiment makes it possible to eliminate a labor required for taking out the wire 3 to improve convenience, and reduce the risk of contamination of the wire 3 to improve safety of the procedure.

Furthermore, the drug solution injection device 100 according to the first embodiment includes the needle catheter 2 having the drug solution lumen 10L, the wire 3 for discharging the drug solution DS in the drug solution lumen 10L from the distal end of the puncture needle 12 (distal end of the needle catheter 2), and the wire delivery device 1 (Fig. 1). Thus, the volume by which the drug solution DS is discharged from the distal end of the puncture needle 12 can be the same as the volume by which the wire 3 is delivered into the drug solution lumen 10L from the wire delivery device 1, so that the injection dose of the drug solution DS can be precisely controlled. In the wire delivery device 1 according to the first embodiment, the drug solution DS in the drug solution lumen 10L can be thoroughly used by extruding the drug solution DS by the wire 3, so that waste of the drug solution DS can be reduced. Furthermore, the wire delivery device 1 discharges the drug solution DS in a volume corresponding to the advanced length of the wire 3, from the distal end of the puncture needle 12 (distal end of the needle catheter 2). Thereby, the injection dose of the drug solution DS can be precisely controlled and waste of the drug solution DS can be reduced. In addition, since the needle catheter 2 has the puncture needle 12, the puncture needle 12 can be used to puncture a living tissue and inject the drug solution DS into the living tissue.

Furthermore, in the drug solution injection device 100 according to the first embodiment, the wire 3 has the first engagement portion 301 on the proximal end portion. The accommodation member 4 for accommodating the wire 3 has the second engagement portion 431 that engages with the first engagement portion 301 on the distal end portion to restrict the movement of the wire 3 in the distal end direction D1. Thus, the wire 3 is sequentially delivered by the wire delivery device 1, and once the proximal end portion (first engagement portion 301) of the wire 3 reaches the distal end portion (second engagement portion 431) of the accommodation member 4, the first engagement portion 301 can engage with the second engagement portion 431 to restrict the movement of the wire 3 in the distal end direction D1 (i.e. stop the delivery of the wire 3) as illustrated in Fig. 7. As a result, the wire 3 can be prevented from falling out of the accommodation member 4, and convenience and safety can be improved.

Furthermore, in the drug solution injection device 100 according to the first embodiment, each transverse sectional shape of the drug solution lumen 10L of the needle catheter 2 and the wire 3 is almost circular, and the outer diameter Φ2 of the wire 3 is 80% or larger of the inner diameter Φ1 of the drug solution lumen 10L (Fig. 2, Fig. 3). Thereby, the drug solution DS in the drug solution lumen 10L can be extruded by the wire 3 while maintaining slidability of the wire 3 in the drug solution lumen 10L. Furthermore, since the main body 50 of the wire delivery device 1 includes the sealing member 57 disposed on the opening 514, the drug solution DS charged in the needle catheter 2 can be prevented from entering the inside of the main body 50, and the main body 50 can be maintained in airtight state.

### <Second Embodiment>

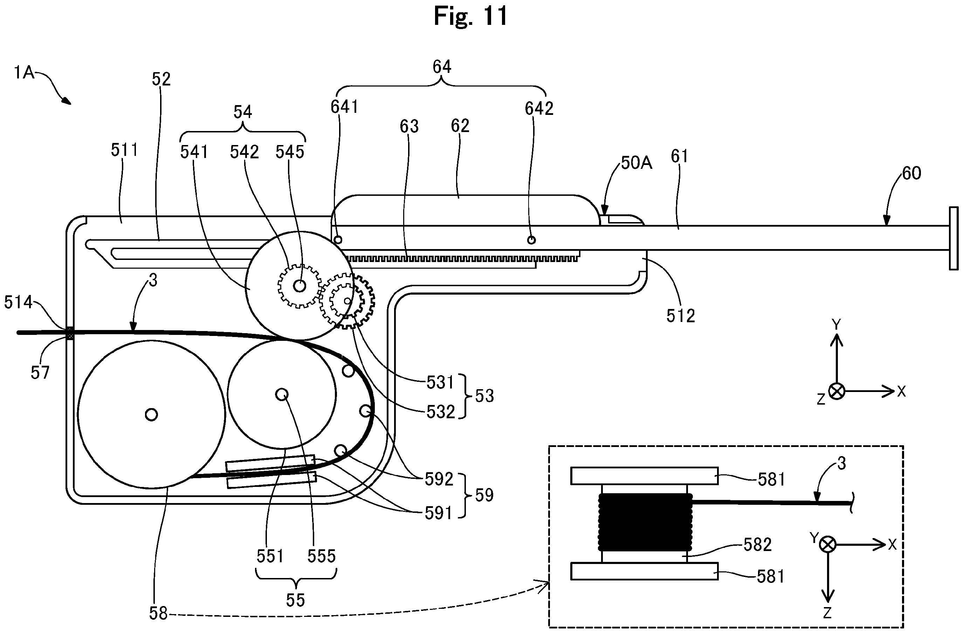

Fig. 11 is an explanatory diagram illustrating a configuration of a wire delivery device 1A according to the second embodiment. A configuration of a wire winding portion 58 is illustrated in the lower part of Fig. 11 (within the dashed frame). The wire delivery device 1A according to the second embodiment includes a main body 50A instead of the main body 50 in the configuration of the first embodiment, and further includes the wire winding portion 58 and a guiding portion 59. The main body 50A does not have the opening 513 and the connector 56 described in the first embodiment.

As illustrated in the lower part of Fig. 11, the wire winding portion 58 has a columnar trunk portion 582, and a pair of flange portions 581 disposed on both ends of the trunk portion 582. The flange portions 581 are disk-shaped with a larger diameter than of the trunk portion 582. The proximal end portion of the wire 3 is fixed to the trunk portion 582, around which a part on the proximal end side of the wire 3 is wound. As illustrated in the upper part of Fig. 11, the guiding portion 59 has guiding walls 591 and three guiding columns 592. The guiding walls 591 are a pair of walls disposed on the inner surface of the main body 50A. The wire 3 is inserted between the walls as illustrated in the figure, and then the guiding walls 591 guide the wire 3 to between the first roller 54 and the second roller 55. The guiding columns 592 are column-shaped members disposed on the inner surface of the main body 50A. The wire 3 is brought into contact with the side surfaces of the guiding columns 592 as illustrated in the figure, and thereby the guiding columns 592 guide the wire 3 to between the first roller 54 and the second roller 55.

In this way, the configuration of the wire delivery device 1A can be modified in various ways. The wire delivery device 1A may be configured so as to have the wire winding portion 58 to accommodate the wire 3. In the wire delivery device 1A, either or both of the guiding walls 591 and the guiding columns 592 may be omitted. The shape of the guiding portion 59 can be optionally changed. For example, curved guiding walls 591 can be used. The same effect as in the first embodiment can also be exhibited by the drug solution injection device 100 using the wire delivery device 1A according to the second embodiment. Since the wire delivery device 1A according to the second embodiment includes the wire winding portion 58 capable of winding up the wire 3, the wire 3 can be wound around and accommodated in the wire winding portion 58 to improve convenience.

### <Third Embodiment>

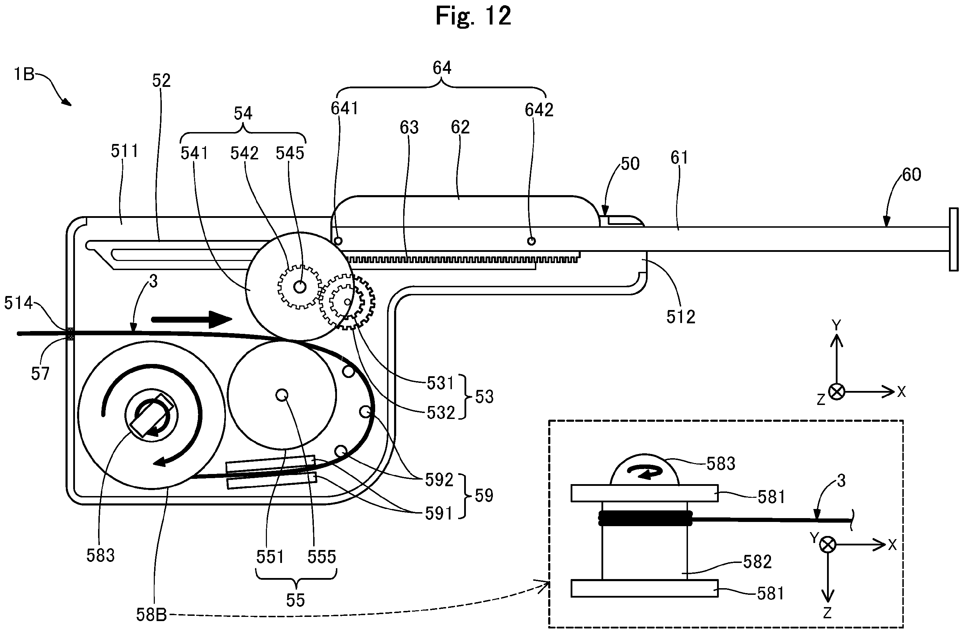

Fig. 12 is an explanatory diagram illustrating a configuration of a wire delivery device 1B according to the third embodiment. The wire delivery device 1B according to the third embodiment includes a wire winding portion 58B instead of the wire winding portion 58 in the configuration of the second embodiment. In the wire winding portion 58B, a knob portion 583 protruding in the +Y-axis direction is disposed on a top surface (surface in the +Y-axis direction) of the flange portion 581. In the wire delivery device 1B, the wire 3 is delivered to the proximal end portion by the manipulation described in Fig. 8 to Fig. 10, then the knob portion 583 is rotated as illustrated in the figure to wind the wire 3 around the wire winding portion 58B.

In this way, the configuration of the wire delivery device 1B can be modified in various ways. In a case of providing the wire winding portion 58B, a constituent for winding up the wire 3 may be further provided. For winding the wire 3, various configurations can be adopted. For example, instead of the knob portion 583 described above, the wire winding portion 58B may incorporate an electric motor and have a switch for switching on/off of the electric motor. The same effect as in the first and second embodiments can also be exhibited by the drug solution injection device 100 using the wire delivery device 1B according to the third embodiment. In addition, the wire delivery device 1B according to the third embodiment makes it easy to wind the wire 3 around the wire winding portion 58B.

### <Fourth Embodiment>

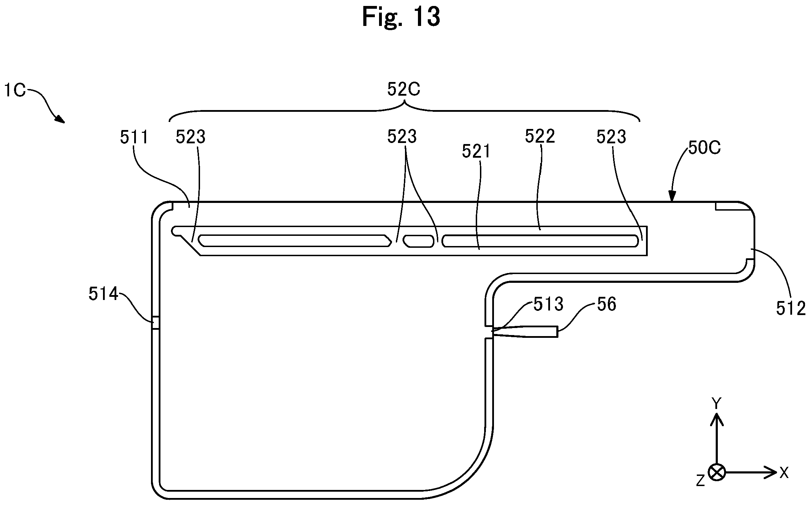

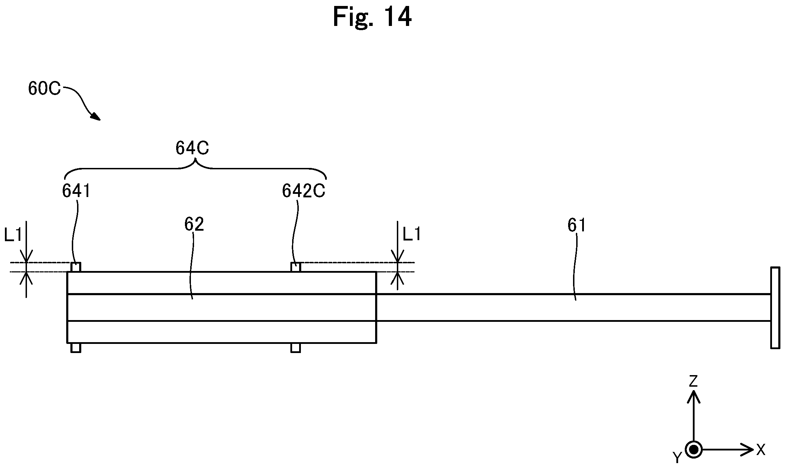

Fig. 13 is an explanatory diagram illustrating a configuration of a main body 50C according to the fourth embodiment. Fig. 14 is an explanatory diagram illustrating a configuration of a rod 60C according to the fourth embodiment. A wire delivery device 1C according to the fourth embodiment includes the main body 50C instead of the main body 50 and the rod 60C instead of the rod 60 in the configuration of the first embodiment. The main body 50C is configured in the same manner as in the first embodiment except that a groove portion 52C is formed instead of the groove portion 52. The groove portion 52C does not have the groove portion 52a on the distal end side and the groove portion 52b on the proximal end side which have different depths, but has a first groove portion 521 and a second groove portion 522 which have the substantially same depth on the distal and proximal end sides, and connection portions 523. The rod 60C is configured in the same manner as in the first embodiment except that a protruding portion 64C is provided instead of the protruding portion 64. In the protruding portion 64C, lengths L1 of the distal end-side protruding portion 641 and a distal end-side protruding portion 641C are the substantially same.

In this way, the configuration of the wire delivery device 1C can be modified in various ways. The wire delivery device 1C may be configured so as to include the groove portion 52C having a constant depth, and the protruding portion 64C having the length L1 matched to the depth of the groove portion 52C. The same effect as in the first embodiment can also be exhibited by the drug solution injection device 100 using the wire delivery device 1C according to the fourth embodiment. In addition, the wire delivery device 1C according to the fourth embodiment can simplify the configuration of the wire delivery device 1C.

### <Fifth Embodiment>

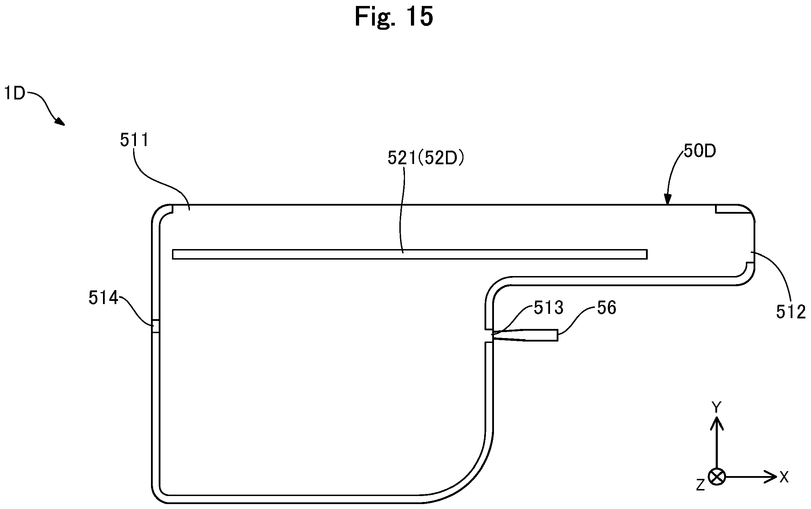

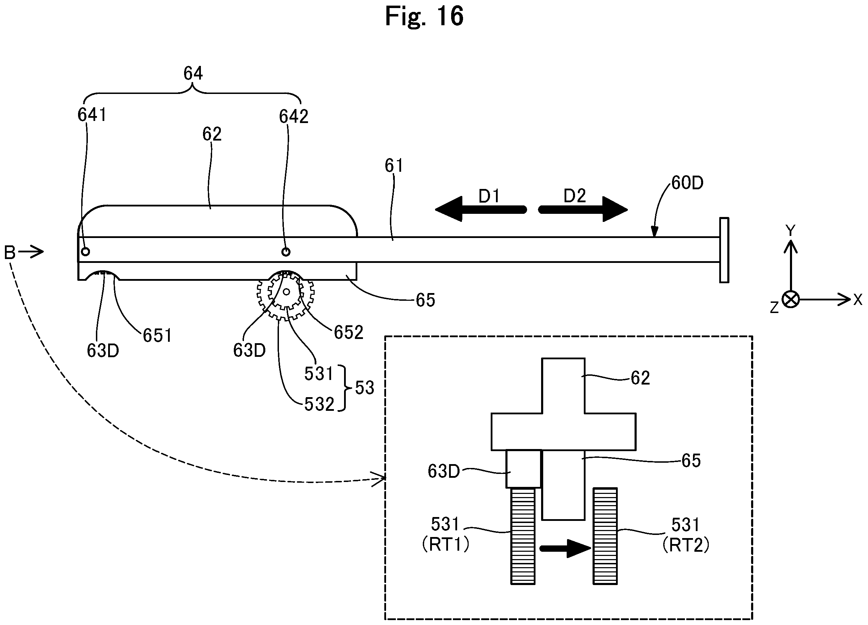

Fig. 15 is an explanatory diagram illustrating a configuration of a main body 50D according to the fifth embodiment. Fig. 16 is an explanatory diagram illustrating a configuration of a rod 60D according to the fifth embodiment. In the lower part of Fig. 16, the rod 60D and the first gear 531 viewed from a direction B in the upper part of Fig. 16 are illustrated. A wire delivery device 1D according to the fifth embodiment includes the main body 50D instead of the main body 50 and the rod 60D instead of the rod 60 in the configuration of the first embodiment.

As illustrated in Fig. 15, the main body 50D is configured in the same manner as in the first embodiment except that a groove portion 52D is formed instead of the groove portion 52. The groove portion 52D does not have the second groove portions 522 and the connection portions 523 described in the first embodiment. As illustrated in the upper part of Fig. 16, the rod 60D includes a compartment portion 65 on a side wall opposite to a side having a projection portion 62 in a rod main body 61. The compartment portion 65 is a long and plate-shaped member that extends in the longitudinal direction (X-axis direction) of the wire delivery device 1D. The compartment portion 65 has arcuate notches 651 and 652 on the distal end side and the proximal end side respectively. As illustrated in the lower part of Fig. 16, a concave/convex portion 63D is disposed on one side of the compartment portion 65. Similarly to the first embodiment, the concave/convex portion 63D is a long and teeth-shaped member that extends in the longitudinal direction (X-axis direction) of the wire delivery device 1D.

In the wire delivery device 1D, the rod 60D is used as follows. Specifically, the operator pushes the rod 60D in the +Z-axis direction to move the first gear 531 from the notch 652 on the proximal end side to the one side of the compartment portion 65 (i.e. side where the concave/convex portion 63D is disposed). In this state, the rod 60D can be moved in the distal end direction D1 to construct the first path RT1 through which the rod 60D moves while the concave/convex portion 63D engages with the gear 53 (first gear 531). Then, the operator draws back the rod 60D in the -Z-axis direction to move the first gear 531 from the notch 651 on the distal end side to the other side of the compartment portion 65, i.e. side where the concave/convex portion 63D is not disposed. In this state, the rod 60D can be moved in the proximal end direction D2 to construct the second path RT2 through which the rod 60D moves while the concave/convex portion 63D does not engages with the gear 53 (first gear 531).

In this way, the configuration of the wire delivery device 1D can be modified in various ways. The first path RT1 and the second path RT2 may be constructed without using the second groove portion 522 and the connection portion 523. Although it is described that the first path RT1 and second path RT2 can be constructed by the rod 60D having the compartment portion 65 and the notches 651 and 652 in the wire delivery device 1D, the first path RT1 and second path RT2 may be constructed by other constituents (e.g. latch gear). The same effect as in the first embodiment can also be exhibited by the drug solution injection device 100 using the wire delivery device 1D according to the fifth embodiment.

### <Sixth Embodiment>

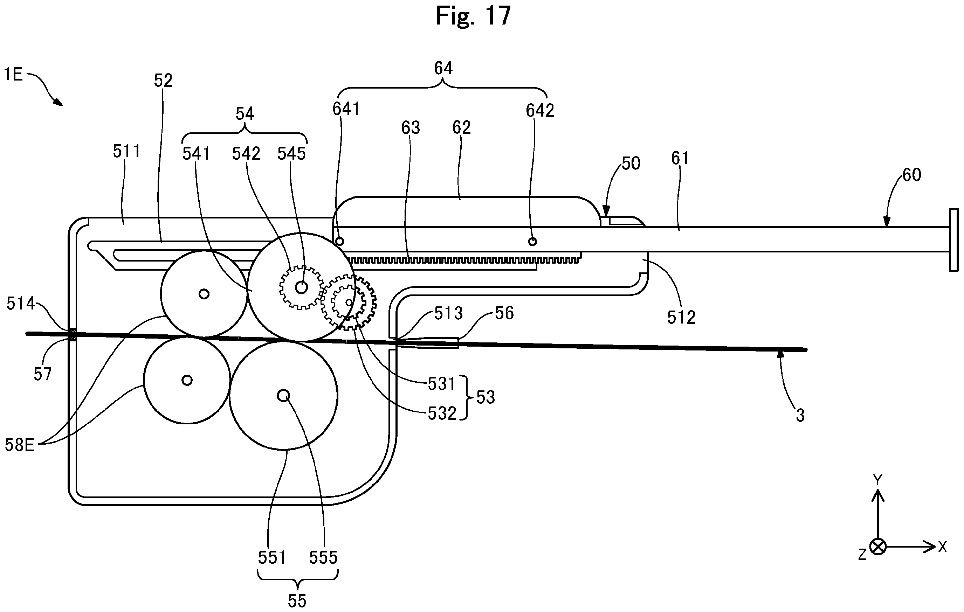

Fig. 17 is an explanatory diagram illustrating a configuration of a wire delivery device 1E according to the sixth embodiment. The wire delivery device 1E further includes a pair of assisting rollers 58E in the configuration of the first embodiment. Each of the assisting rollers 58E is almost hollow cylindrical or almost columnar. The assisting rollers 58E are disposed between the first roller 54/second roller 55 and the opening 514 to sandwich the wire 3. The assisting rollers 58E rotate in response to the rotation of the first roller 54 and the second roller 55 to deliver the wire 3 in the distal end direction D1. Thus, the configuration of the wire delivery device 1E can be modified in various ways. Other constituents for assisting the function of the wire delivery device 1E, such as the assisting rollers 58E may be provided. The same effect as in the first embodiment can also be exhibited by the drug solution injection device 100 using the wire delivery device 1E according to the sixth embodiment.

### <Seventh Embodiment>

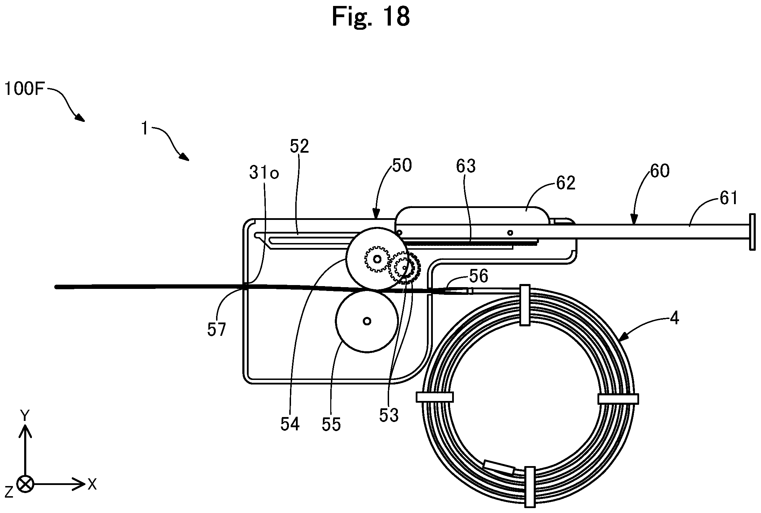

Fig. 18 is an explanatory diagram illustrating a configuration of a drug solution injection device 100F according to the seventh embodiment. The drug solution injection device 100F according to the seventh embodiment does not have the needle catheter 2 in the configuration described in the first embodiment. The wire 3 delivered by the drug solution injection device 100F may be either inserted directly into a living body lumen via a sheath not illustrated, or inserted into a device such as a balloon catheter and a guiding catheter. As described above, the configuration of the drug solution injection device 100F can be modified in various ways. At least some of the aforementioned constituents such as the needle catheter 2 and the accommodation member 4 may be omitted. The same effect as in the first embodiment can also be exhibited by the drug solution injection device 100F according to the seventh embodiment.

### <Eighth Embodiment>

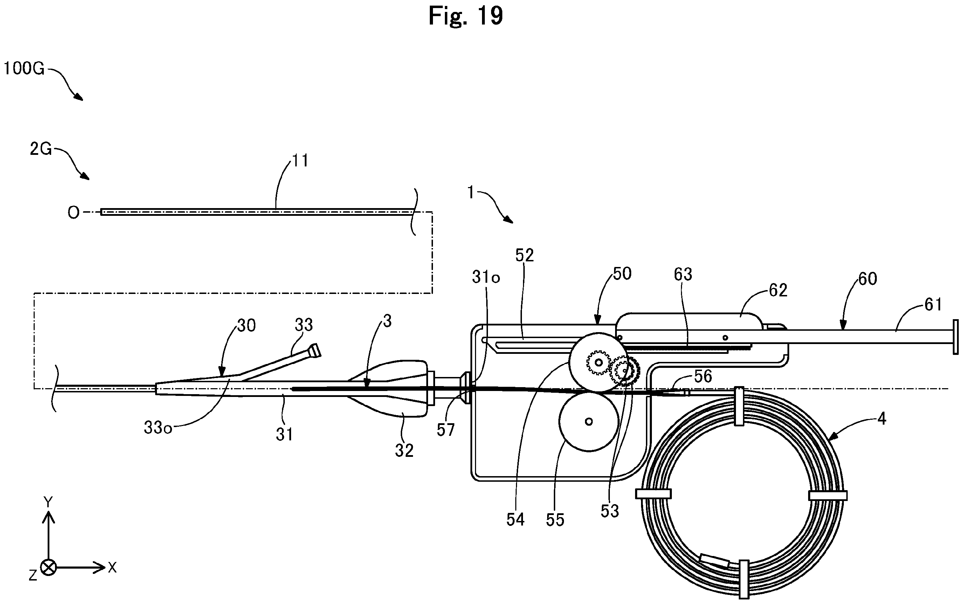

Fig. 19 is an explanatory diagram illustrating a configuration of a drug solution injection device 100G according to the eighth embodiment. The drug solution injection device 100G according to the eighth embodiment has a catheter 2G instead of the needle catheter 2 in the configuration described in the first embodiment. The catheter 2G does not have the puncture needle 12, and the drug solution is discharged from the opening on the distal end portion of the hollow shaft 11. As described above, the configuration of the drug solution injection device 100G can be modified in various ways. The catheter 2G without having the puncture needle 12 may be provided. The same effect as in the first embodiment can also be exhibited by the drug solution injection device 100G according to the eighth embodiment. The drug solution injection device 100G according to the eighth embodiment makes it possible to thoroughly use the drug solution in the drug solution lumen 10L even in a case without requiring puncture, e.g. a case that the drug solution is intended to be applied to a surface of a living tissue, so that waste of the drug solution can be reduced.

### <Variants of Embodiments>

The present invention is not limited to the above-described embodiments, and may be implemented in various modes without departing from the spirit of the present invention. The following modifications can be applied, for example.

### [Variant 1]

In the first to eighth embodiments, some examples of the configurations of the drug solution injection devices 100, 100F, and 100G have been described. However, the configuration of the drug solution injection device 100 can be modified in various ways. For example, the drug solution injection device 100 may include a compartment plate for dividing the interior of the main body 50, an auxiliary for assisting the insertion of the wire 3 into the first roller 54 and the second roller 55, and a fixture for fixing and holding the accommodation member 4 attached to the connector 56, or the like. For example, the drug solution injection device 100 may include a control unit for controlling each portion of the drug solution injection device 100. For example, the control unit may measure and display the movement amount of the rod 60 (or delivery amount of the wire 3).

### [Variant 2]

In the first to eighth embodiments, some examples of the configurations of the needle catheter 2 and the catheter 2G have been described. However, the configurations of the needle catheter 2 and the catheter 2G can be modified in various ways. For example, the needle catheter 2 and the catheter 2G may be configured as a multi-lumen catheter including another lumen different from the drug solution lumen 10L for the drug solution. For example, a valve member for restricting reverse flow of the drug solution (movement toward the wire insertion port 31o side or the drug solution feed port 33o side) may be disposed in the drug solution lumen 10L of the needle catheter 2 or the catheter 2G. For example, the needle catheter 2 and the catheter 2G may include a mesh member for positioning the distal end portion of the catheter in the living body lumen. For example, the needle catheter 2 and the catheter 2G may include a balloon member for intercepting current of body fluids (e.g. blood) flowing through the living body lumen and positioning the distal end portion of the catheter.

For example, the drug solution feed port 33o and the wire insertion port 31o of the connector 30 may be disposed at the substantially same position in the axis line O direction. For example, in the axis line O direction, the drug solution feed port 33o of the connector 30 may be disposed on the proximal end side with respect to the wire insertion port 31o. For example, the connector 30 need not include the second extension portion 33 having the drug solution feed port 33o. In this case, the operator may perform an operation in which a syringe is attached to the wire insertion port 31o to charge the drug solution into the connector 30, then the syringe is removed and the wire delivery device 1 is attached to the same wire insertion port 31o to deliver the wire 3.

### [Variant 3]

In the first to eighth embodiments, some examples of the configurations of the wire delivery devices 1 and 1A to 1E have been described. However, the configuration of the wire delivery device 1 can be modified in various ways. For example, for the wire delivery device 1, a configuration that an electric rod is moved using an electric motor or the like may be adopted instead of the rod 60 that is manually moved in the distal end direction D1 or the proximal end direction D2. In this case, the wire delivery device 1 may further include an input portion for inputting a dose of the drug solution (or movement amount of the electric rod). The input portion can be constructed in any form such as a touch panel or operation buttons. For example, the wire delivery device 1 may include a fixation member for fixing the wire delivery device 1 to the wire insertion port 31o of the connector 30.

For example, the wire delivery device 1 may transmit a rotative force generated from the rod 60 by using a means other than the rack-and-pinion mechanism. As the means other than the rack-and-pinion mechanism, for example, pulley and belt, magnetic force, or the like can be used. For example, the first gear 531, the second gear 532, and the shaft 535 of the gear 53 may be integrally formed. The above description also applies to the first roller 54.

### [Variant 4]

In the first to eighth embodiments, some examples of the configurations of the wire 3 and the accommodation member 4 have been described. However, the configurations of the wire 3 and the accommodation member 4 can be modified in various ways. For example, the outer diameter of the wire 3 need not be substantially constant from the distal end to the proximal end. In this case, the wire 3 may be configured such that a part on the distal end side is formed so as to have a relatively small diameter, and a part on the proximal end side is formed so as to have a relatively large diameter. For example, the outer diameter of the wire 3 may be less than 80% of the inner diameter of the drug solution lumen 10L. For example, the transverse sectional shape of the wire 3 need not be almost circular. For example, the first engagement portion 301 is not necessarily disposed on the proximal end portion of the wire 3. For example, the wire 3 is not necessarily accommodated in the accommodation member 4. For example, the accommodation member 4 need not have the accommodation tube 41, the fasteners 42, and the distal end member 43. The accommodation member 4 may be simply a bag-shaped body.

### [Variant 5]

The configurations of the wire delivery devices 1 and 1A to 1E, the needle catheter 2, the catheter 2G, the wire 3, and the accommodation member 4 in the first to eighth embodiments and Variants 1 to 4 may be appropriately combined. For example, it is allowed to combine the main body 50 and the rod 60 described in the fourth embodiment, or the main body 50 and the rod 60 described in the fifth embodiment, with the wire winding portion 58 and the guiding portion 59 described in the second or third embodiment, and with the assisting rollers 58E described in the sixth embodiment.

The aspects have been described on the basis of embodiments and variants so far, but the embodiments of the aspect described above are provided to facilitate understanding the aspects and not to limit the aspects. The aspects can be altered or modified without departing from the spirit and scope, as well as encompass the equivalents thereof. In addition, the technical features can be appropriately deleted as long as it has not been described as an essential feature herein.

### DESCRIPTION OF REFERENCE NUMERALS

- 1, 1A to 1E: Wire delivery device
- 2: Needle catheter
- 2G: Catheter
- 3: Wire
- 4: Accommodation member
- 10L: Drug solution lumen
- 11: Hollow shaft
- 12: Puncture needle
- 30: Connector
- 31: First extension portion
- 31o: Wire insertion port
- 32: Blade portion
- 33: Second extension portion
- 33o: Drug solution feed port
- 34: First extension portion
- 41: Accommodation tube
- 42: Fastener
- 43: Distal end member
- 50, 50A, 50C, 50D: Main body
- 52, 52C, 52D: Groove portion
- 53: Gear
- 54: First roller
- 55: Second roller
- 56: Connector
- 57: Sealing member
- 58, 58B: Wire winding portion
- 58E: Assisting roller
- 59: Guiding portion
- 60, 60C, 60D: Rod
- 61: Rod main body
- 62: Projection portion
- 63, 63D: Concave/convex portion
- 64, 64C: Protruding portion
- 65: Compartment portion
- 651, 652: Notch
- 100, 100G: Drug solution injection device
- 301: First engagement portion
- 431: Second engagement portion
- 511 to 514: Opening
- 521, 521a, 521b: First groove portion
- 522, 522a, 522b: Second groove portion
- 523, 523a, 523b: Connection portion
- 531: First gear
- 532: Second gear
- 535: Shaft
- 541: Roller portion
- 542: Third gear
- 545: Shaft
- 551: Roller portion
- 555: Shaft
- 581: Flange portion
- 582: Trunk portion
- 583: Knob portion
- 591: Guiding wall
- 592: Guiding column
- 641, 641C: Distal end-side protruding portion
- 642: Proximal end-side protruding portion

## Claims

1. A wire delivery device for delivering a wire, the wire delivery device comprising:
a rod that has a long external shape and a side surface with concaves/convexes arranged along a longitudinal direction and is capable of moving in a distal end direction of the wire delivery device;
a gear that engages with the concaves/convexes of the rod and rotates in response to the movement of the rod in the distal end direction; and
a first roller and a second roller that sandwich the wire therebetween and individually rotate in response to the rotation of the gear to deliver the wire in the distal end direction of the wire delivery device.

2. The wire delivery device according to claim 1, wherein
the rod is capable of:
moving not only in the distal end direction of the wire delivery device but also in a proximal end direction of the wire delivery device;
moving with the concaves/convexes engaging with the gear in a first path in the distal end direction; and
moving with the concaves/convexes not engaging with the gear in a second path in the proximal end direction.

3. The wire delivery device according to claim 2, further comprising a main body that accommodates a part on a distal end side of the rod, the gear, and the first roller and the second roller, wherein
the main body has an inner surface having:
a first groove portion for the first path,
a second groove portion for the second path, and
a connection portion for connecting the first groove portion with the second groove portion, and
the rod further has a protruding portion protruding on the side surface, and
moves through the first path while the protruding portion engages with the first groove portion and moves through the second path while the protruding portion engages with the second groove portion.

4. The wire delivery device according to any one of claims 1 to 3, further comprising a main body that accommodates a part on a distal end side of the rod, the gear, and the first roller and the second roller, the main body having a connector that is attachable with a distal end portion of an accommodation member for the wire.

5. The wire delivery device according to any one of claims 1 to 3, further comprising a wire winding portion capable of winding up the wire while fixing a part on the proximal end side of the wire.

6. A drug solution injection device comprising:
a catheter having a hollow first main body with a long external shape, a drug solution lumen that is formed inside the first main body to contain a drug solution;
a wire that is inserted into the drug solution lumen and advanced from a proximal end toward a distal end of the drug solution lumen to discharge the drug solution in the drug solution lumen from a distal end of the catheter; and
the wire delivery device according to any one of claims 1 to 5 that delivers the wire.

7. The drug solution injection device according to claim 6, wherein
the wire has a first engagement portion on a proximal end portion and is accommodated in an accommodation member, and
the accommodation member has a second engagement portion that engages with the first engagement portion on a distal end portion to restrict movement of the wire in a distal end direction.

8. The drug solution injection device according to claim 6 or 7, wherein
the wire has an outer diameter substantially constant from the distal end to a proximal end, and
the wire delivery device advances the wire inside the drug solution lumen to discharge the drug solution in a volume corresponding to an advanced length of the wire, from the distal end of the catheter.

9. The drug solution injection device according to any one of claims 6 to 8, wherein
the catheter is a needle catheter that further has a hollow puncture needle disposed on a distal end portion of the first main body, and
the wire discharges the drug solution in the drug solution lumen from a distal end of the puncture needle.
